# EUROPEAN PATENT APPLICATION

(11) **EP 0 561 296 A1**
(43) Date of publication of application: **22.09.1993**
(21) Application number: 93104061.2
(22) Date of filing: 12.03.1993
(51) Int. Cl.: C07F 9/6503, A61K 31/675, C07F 9/6509, C07J 51/00, A61K 31/565

(54) **Cyclic aminomethylene bisphosphonate derivatives**

(30) Priority: 18.03.1992 JP 61735/92
(71) Applicant: HOECHST JAPAN LIMITED, Tokyo 100-91 (JP)
(72) Inventor: Sugioka, Tatsuo, Iruma-gun, Saitama (JP); Satoh, Ryoichi, Tsurugashima, Saitama (JP); Kitamura, Kazuyuki, Sakodi-shi, Saitama (JP)
(74) Representative: Fischer, Hans-Jürgen, Dr.

(57) **Abstract**

A compound represented by the formula (I) 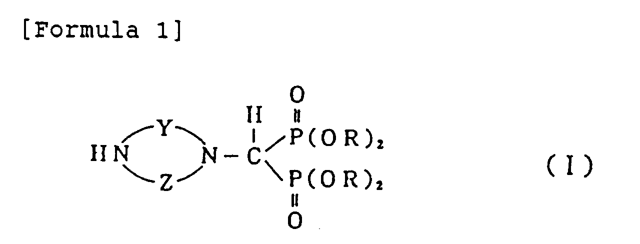
or the formula (II) 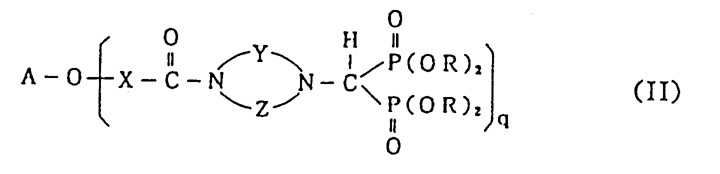
wherein R denotes a hydrogen atom or a lower alkyl group; Y and Z may be the same or different and independently denote alkylene groups containing 2-5 carbon atoms in their alkylene chains which may be substituted with a lower alkyl(s); X denotes a single bond or an alkylene group containing 1-10 carbon atoms in its alkylene chain which may be substituted with a lower alkyl(s); A-O- denotes a residue of a compound having an estrogenic activity and q denotes an integer of 1-3, and physiologically acceptable salts thereof.

The above compounds (I) or (II) are useful as a bone resorption inhibitor and a therapeutic agent for bone diseases.

## Description

### 1. Field of the Invention

This invention relates to novel cyclic aminomethylene bisphosphonate derivatives and physiologically acceptable salts thereof. They are useful as a bone resorption inhibitor and a therapeutic agent for bone diseases.

### 2. Description of the Prior Art

Therapeutic agents for bone diseases such as estrogen, calcitonins, vitamin D₃ and derivatives thereof, ipriflavone and bisphosphonate derivatives are currently available.

Among these agents, the bisphosphonate derivatives, which are known as bone resorption inhibitors, have a high affinity to bone tissues and are hardly metabolized and their possible uses as a drug carrier are reported (Bone, vol. 8, suppl. 1, p. 23-28, 1987).

While estrogen is therapeutically useful in bone diseases, there are pointed out risks of more or less increased occurrence of intrauterine hemorrhage and endometrial cancer ("IGAKUNOAYUMI (Development of Medical Science)", p. 749, vol. 152, No. 12, 1990). It is therefore desirable to selectively deliver estrogen to the affected part of bone thereby reducing the possible adverse reactions.

It is an object of the invention to provide novel cyclic aminomethylene bisphosphonate derivatives and salts thereof and novel cyclic methylene bisphosphonate-estrogen compounds in which the bisphosphonate compound is coupled with an estrogen having a hydroxyl group in the molecule through a spacer.

It is another object of the invention to provide a pharmaceutical composition for inhibiting a bone resorption containing an effective amount of the above compounds.

It is a further object of the invention to provide a pharmaceutical composition for treating bone diseases containing an effective amount of the above compounds.

It is a further object of the invention to provide a method for inhibiting a bone resorption containing an effective amount of the above compounds.

It is a further object of the invention to provide a method for treating bone diseases containing an effective amount of the above compounds.

The above-mentioned cyclic aminomethylene bisphosphonate derivatives are used as a bone resorption inhibitor. It is also expected that the cyclic aminomethylene bisphosphonate-estrogen compounds are selectively incorporated into bone tissues and the compounds themselves or their metabolites inhibit bone resorption and also exhibit a positive therapeutic effect in other bone diseases.

According to the invention, there is provided a compound represented by the formula (I) 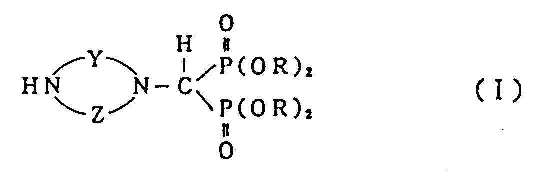
or the formula (II) 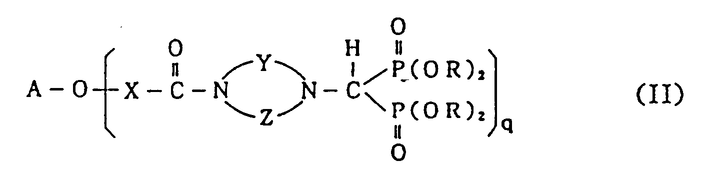
wherein R denotes a hydrogen atom or a lower alkyl group; Y and Z may be the same or different and independently denote alkylene groups containing 2-5 carbon atoms in their alkylene chain which may be substituted with a lower alkyl(s); X denotes a single bond or an alkylene group containing 1-10 carbon atoms in their alkylene chains which may be substituted with a lower alkyl(s); A-O- denotes a residue of a compound having an estrogenic activity and q denotes an integer of 1-3, and physiologically acceptable salts thereof.

A preferred compound of the formula (I) in the invention is one having the formula (III) 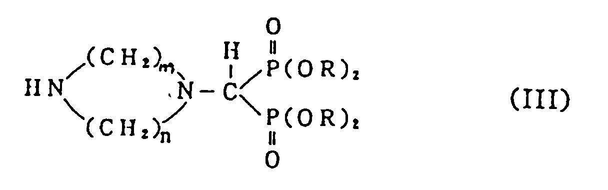
wherein R has the same meaning as defined above and m and n which may be the same or different denote integers of 2 or 3, and physiologically acceptable salts thereof. An especially preferred compound is one wherein both m and n are 2, and physiologically acceptable salts thereof.

A preferred compound of the formula (II) in the invention is one having the formula (IV) 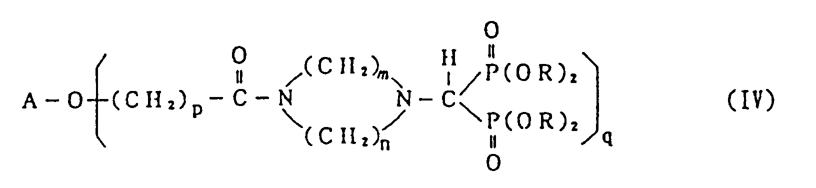
wherein A-O-, R, q, m and n have the same meanings as defined above and p denotes an integer of 0-5, and physiologically acceptable salts thereof. The compounds wherein p is 0 or 1, and both m and n are 2 and physiologically acceptable salts thereof are especially preferable.

The lower alkyl group for R in the formulae (I)-(IV) means an alkyl group containing 1-6 carbon atoms. As a preferred lower alkyl group is mentioned a straight or branched chain lower alkyl, such as methyl, ethyl, propyl, isopropyl, butyl, isobutyl, tert-butyl, pentyl or hexyl.

The alkylene group for X may be straight or branched and includes methylene, ethylene, trimethylene, n-butylene, isobutylene, tert-butylene, n-pentylene, neopentylene, n-hexylene, n-heptylene, n-octylene, n-nonylene, n-decalene.

The lower alkyl(s) substituted to Y, Z and X means an alkyl group containing 1-4 carbon atoms. As a preferred lower alkyl group is mentioned a straight or branched chain lower alkyl such as methyl, ethyl, n-propyl, isopropyl, n-butyl, sec-butyl or tert-butyl.

The compound the residue in which is represented by A-O- in the formulae (II) and (IV) includes those having a hydroxyl group in their molecules and possessing an estrogenic activity, for example, natural estrogens such as estron, estradiol and estriol and derivatives thereof; plant estrogens including isoflavon derivatives known to be a nonsteroid estrogen such as genistein, biochanin-A, formononetin and daidzein and miroestrol; as well as synthetic estrogens such as stilbestrol, hexestrol and benzestrol or derivatives thereof.

Typical examples of the group A-O- are listed below. 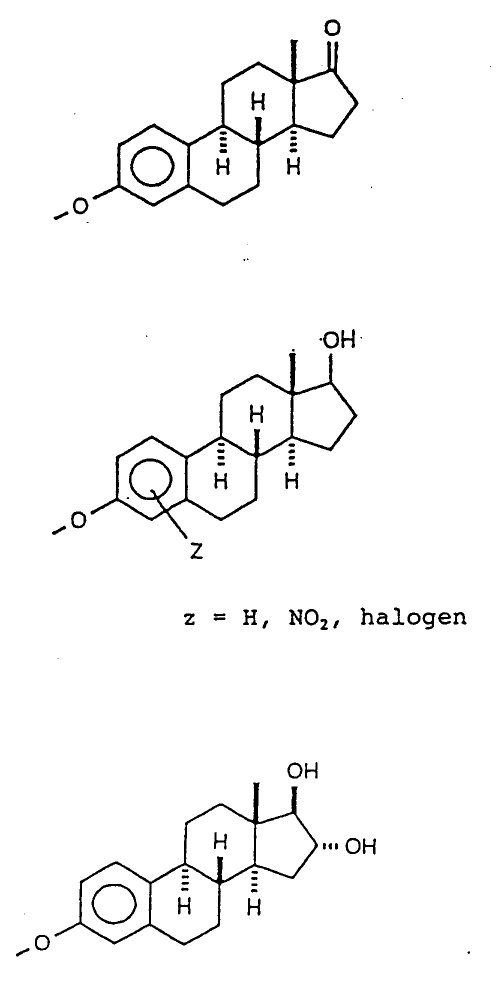 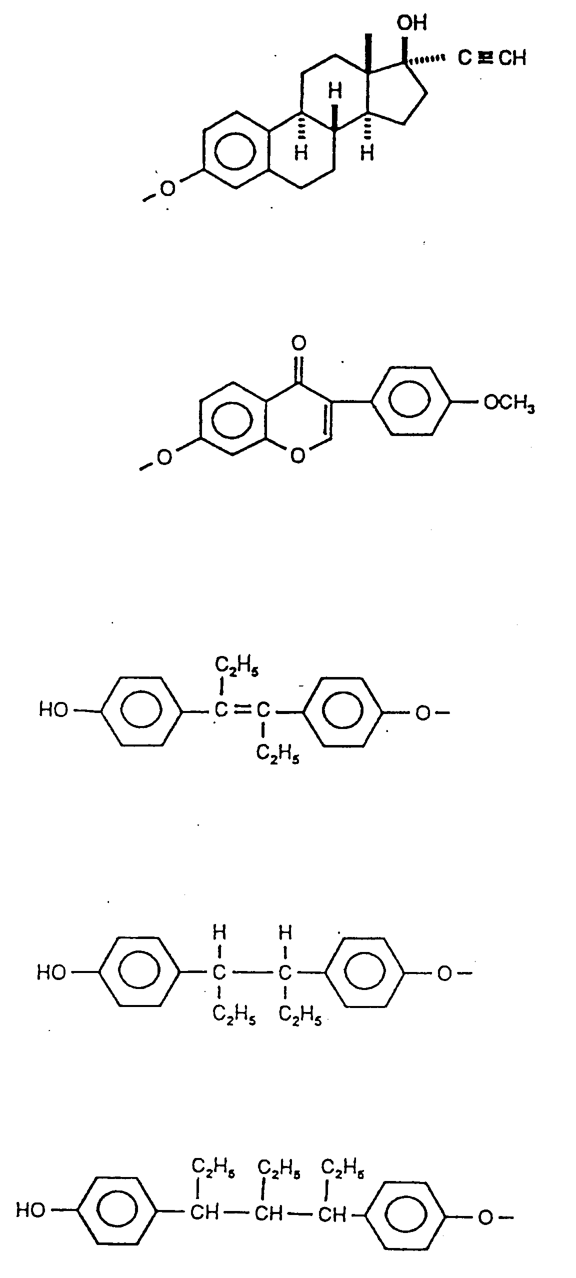 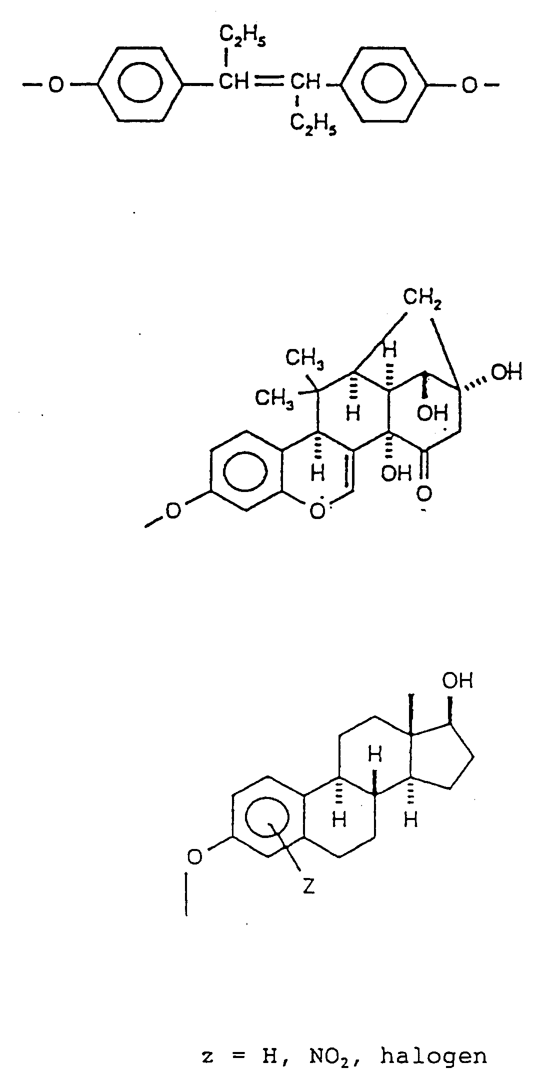 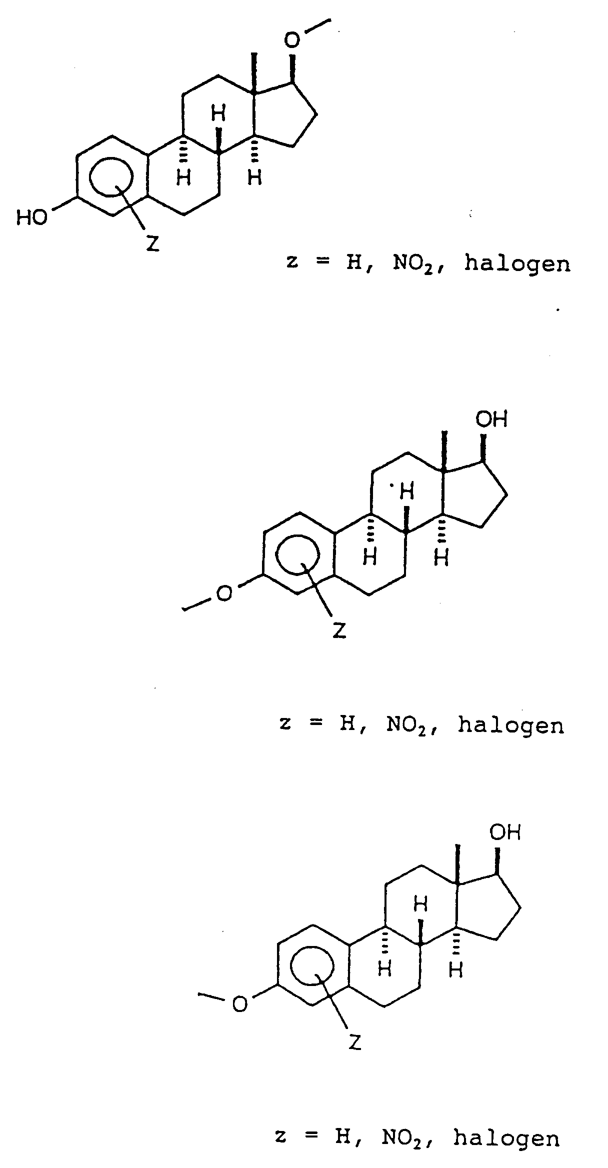
The physiologically acceptable salts of a compound of the formulae (I)-(IV) may be conventional nontoxic salts, examples of which are mentioned salts of the compound with an alkali metal such as sodium or potassium, an alkaline earth metal such as calcium or magnesium, or an inorganic base such as aluminum, or an organic amine such as methylamine, ethylamine, propylamine, isopropylamine, butylamine, tert-butylamine, dimethylamine, diethylamine, trimethylamine, triethylamine, pyridine, picoline, dicyclohexylamine or N,N'-dicyclohexylamine.

A process for the preparation of the cyclic aminomethylene bisphosphonate derivatives (I)-(IV) may be shown by the following reaction schemes: 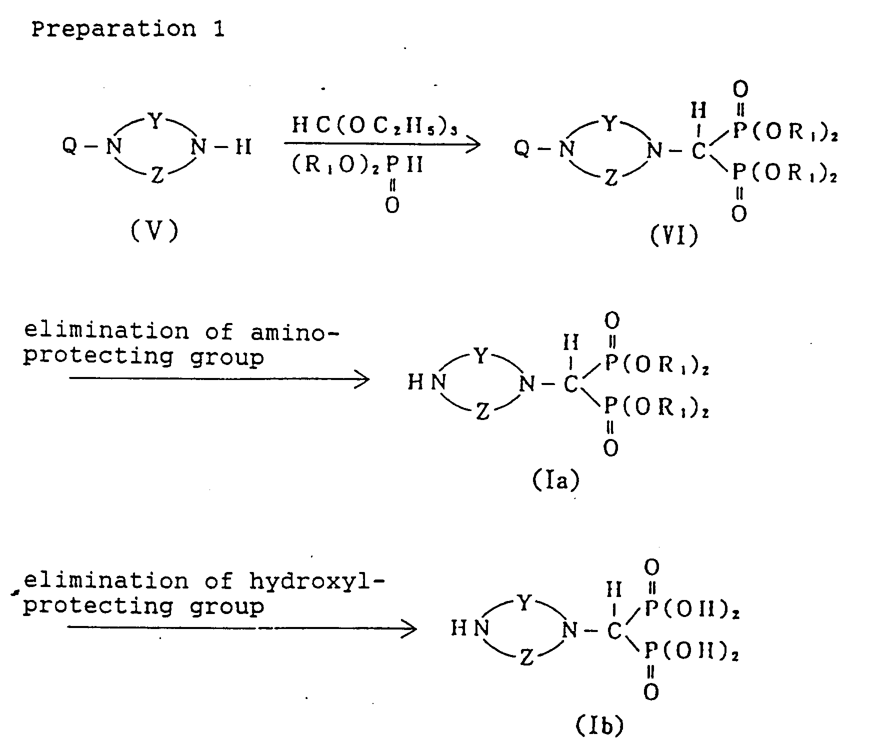
wherein Q denotes an amino-protecting group; R₁ denotes a lower alkyl group, and Y and Z have the same meanings as defined above. 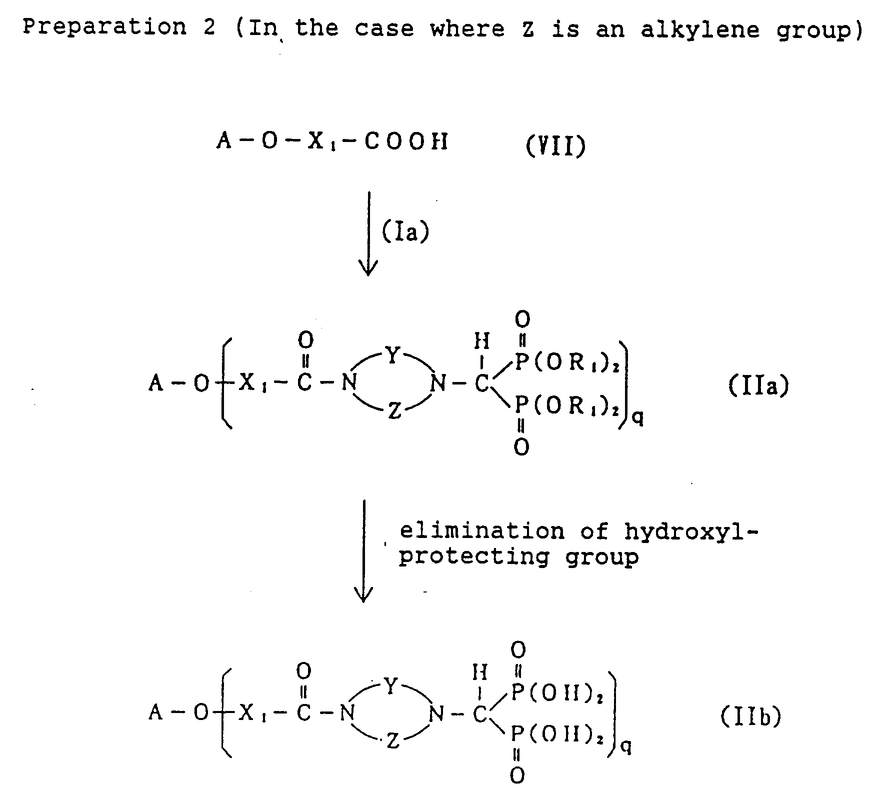
wherein X₁ denotes an alkylene group containing 1-10 carbon atoms in its alkylene chain which may be substituted with a lower alkyl group and A-O-, Y, Z and R₁ have the same meanings as defined above. 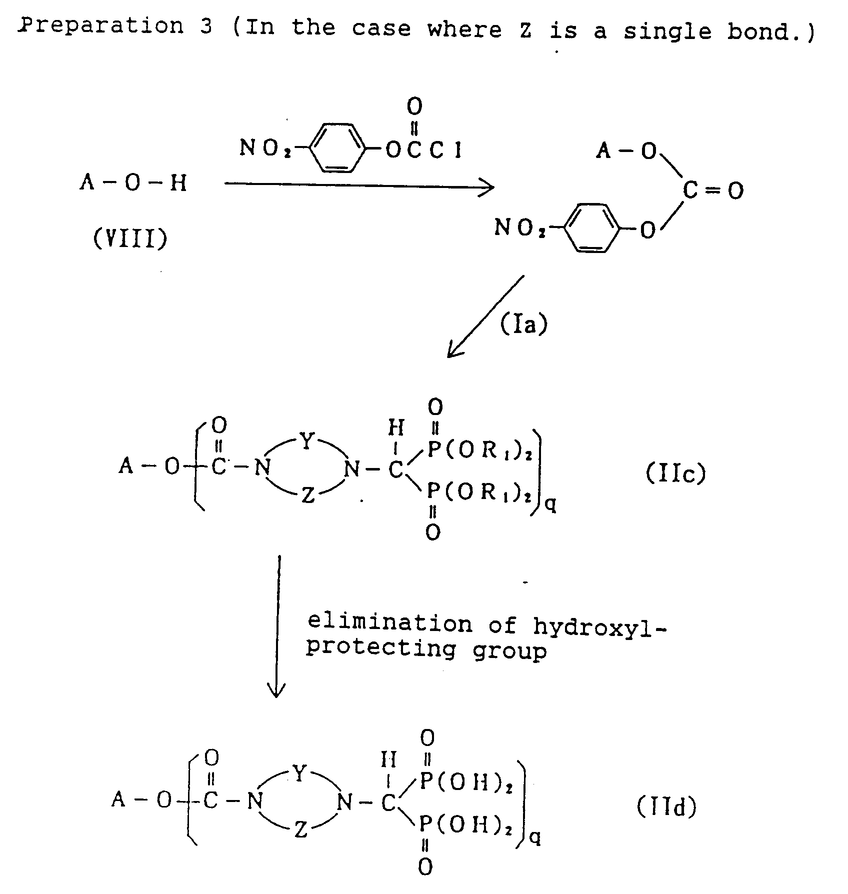
wherein A-O-, Y, Z, q and R₁ have the same meanings as defined above.

The preparations 1-3 will be described in detail below.

### Preparation 1

A compound (V) is reacted with a mixture of triethyl orthoformate and a dialkyl phosphite to give a compound (VI). The compound (VI) is converted by elimination of an amino-protecting group to a compound (Ia), which is subjected, if necessary, to elimination of a hydroxyl-protecting group to afford compound (Ib).

The method for converting the compound (V) to the compound (VI) is known per se, and may be performed according to the method described in literatures such as Ger. Offen. 2,831,578 and Phosphorus and Sulfur 1981, vol. 11, pages 311-322.

Q in the compounds (V) and (VI) is an amino-protecting group. As examples of the group are mentioned the conventional groups such as benzyl and triphenylmethyl that can be eliminated by catalytic reduction. The compounds (V) are produced by introducing a protecting group into such a compound as piperazine, 2-methylpiperazine, 2-ethylpiperazine, 2,5-dimethylpiperazine, 2,6-dimethylpiperazine or homopiperazine.

The elimination of an amino-protecting group in the compounds (VI) is carried out by a conventional method, preferably by catalytic reduction using an alcohol solvent such as ethanol in the presence of palladium on carbon.

The elimination of a hydroxyl-protecting group in the compounds (Ia) is carried out by a conventional method such as hydrolysis.

The hydrolysis is preferably carried out in the presence of a base, an acid including Lewis acids or a halosilane compound.

As the base are mentioned inorganic bases such as sodium hydroxide, potassium hydroxide and sodium carbonate, as well as organic bases such as trimethylamine and triethylamine.

As the acid are mentioned organic acids such as formic acid, acetic acid and trifluoroacetic acid, as well as, inorganic acids such as hydrochloric acid and hydrobromic acid.

The halosilane compound can be exemplified by halotri(lower)alkylsilanes such as iodotrimethylsilane and bromotrimethylsilane. Incidentally, when a Lewis acid such as trihaloacetic acids, e.g. trichloroacetic acid and trifluoroacetic acid, is used in the elimination reaction, the reaction is preferably carried out in the presence of a cation scavenger such as anisole and phenol.

While the reaction is usually carried out in water, an alcohol such as methanol and ethanol, methylene chloride, acetonitrile, chloroform, tetrachloromethane, tetrahydrofuran or a mixture of these solvents, the reaction can be carried out in any other solvents so long as they do not adversely affect the reaction. Incidentally, a liquid base, acid or halosilane compound can be used as the solvent.

While the reaction temperature is not critical, the reaction is usually carried out with cooling or heating.

### Preparation 2

The compounds (IIa) and (IIc) can be synthesized as follows:
Condensation of the compound (VII) with the compound (Ia) yields the compound (IIa). The reaction for producing the compounds (IIa) may be carried out in the presence of a conventional condensing agent such as carbonyldiimidazole or N,N-dicyclohexylcarbodimide. The reaction is carried out in a conventional solvent such as dioxane, tetrahydrofuran, benzene, chloroform, methylene chloride or N,N-dimethylformamide. However, any other solvents may be employed for the reaction so long as they do not adversely affect the reaction.

The reaction may also be conducted in the presence of an organic base such as a tri(lower)alkylamine, pyridine, a di(lower)alkylaminopyridine or an N-(lower)alkylmorphorine.

While the reaction temperature is not critical, the reaction is usually carried out with cooling or heating.

A method for the production of the compound (IIa) from the compound (VII) and the compound (Ia) is not limited to one using a condensing agent. For example, the compounds (IIa) may also be obtained by a method using a reactive derivative on their carboxyl groups such as an acid halogenide or an acid anhydride or a known method for acid amide synthesis using a reactive derivative on their amino groups.

The elimination of a hydroxyl-protecting group of the compounds (IIa) to give the compounds (IIb) or their salts may be conducted in the same way as in Preparation 1.

### Preparation 3

The compounds (IIc) and (IId) can be synthesized as follows:
The compound (VIII) is reacted with 4-nitrophenyl chloroformate to give an active acid anhydride derivative, which is then reacted with the compound (Ia) to afford the compound (IIc).

The reaction for producing the compound (IIc) which has a carbamate bond is usually carried out in a conventional solvent such as dioxane, tetrahydrofuran, benzene, chloroform, methylene chloride or N,N-dimethylformamide. However, the reaction may be conducted in any other solvents so long as they do not adversely affect the reaction.

The reaction may also be carried out in the presence of an inorganic base or an organic base such as an alkali metal hydrogen carbonate, a tri(lower)alkylamine, pyridine, a di(lower)alkylaminopyridine or an N-(lower)alkylmorpholine.

The reaction temperature is not critical, but the reaction is usually conducted with cooling or heating.

The active acid anhydride derivative obtained by the reaction with 4-nitrophenyl chloroformate may be used in the next reaction in a form of the reaction mixture. Alternatively, it may be subjected after isolation to a simple purification operation such as column chromatography for next use.

A method for the production of the carbamate bond-containing compound (IIc) from the compound (VIII) and the compound (Ia) is not limited to one using 4-nitrophenyl chloroformate to produce an active acid anhydride derivative. For example, the compound (IIc) may also be produced by a method using phosgen to convert to a chloroformyl derivative or by a known carbamate synthesis in which an amino group is converted to an isocyanate derivative.

The elimination of a hydroxy-protecting group in the compound (IIc) may be carried out in the same way as in Preparation 1 to give the compound (IId) or its salt.

If the desired compounds (I) and (II) have one or more stereo-isomers such as optical isomers and geometrical isomers, based on the asymmetric carbon atom or the double bond present in the molecule, such isomers and mixtures thereof are all included within the scope of the desired compounds (I) and (II).

If the residue A-O- of the compound possessing an estrogenic activity has a plurality of hydroxyl groups, the binding site to the bisphosphonate derivative may not particularly be limited, and those compounds in which all or a particular one of the hydroxyl groups is bound to the bisphosphonate derivative are also included within the scope of the desired compounds (II).

If the compound possessing an estrogenic activity and having a plurality of hydroxyl groups and reactive functional groups such as amine, carboxylic acid and ketone is allowed to react at a particular hydroxyl group with the bisphosphonate derivative, the hydroxyl group and the reactive functional groups are protected with a suitable protective group, respectively, followed by deprotection, to obtain a desired compound (II).

The compound of the invention may usually be administered to an adult at a unit dose of 100 µg to 1,000 mg once per 1 to 15 days, either orally or parenterally such as by intravenous injection, subcutaneous injection or intramuscular injection. The dosage may appropriately be adjusted depending on types of the compound, age, sex, body weight and symptoms of the patient, and administration route.

The compound of the invention is formed into a formulation for oral or parenteral administration using, as necessary, a pharmaceutical carrier or excipient.

Tablets, powders, capsules, granules and other preparations for oral administration may contain conventional excipients such as calcium carbonate, calcium phosphate, corn starch, potato starch, sugar, lactose, talc, magnesium stearate and gum arabic. Liquid preparations for oral administration may be in the form of aqueous or oily suspension, solution, syrup, elixir, etc.

Injections may be in the form of solution or suspension prescribed with such agents as a suspending agent, a stabilizer and a dispersant. They may further comprise sterile distilled water, a purified oil such as peanut oil or corn oil, or a nonaqueous solvent such as polyethylene glycol or polypropylene glycol.

The following examples are intended to illustrate the preparation of the compounds of the invention, but are not to be construed as limiting the scope thereof.

### Example 1

(i) A mixture of 1-benzylpiperazine (10.0g), triethyl orthoformate (10.1 g) and diethyl phosphite (31.3 g) was heated to 150°C and stirred for about 2 hours. After completion of the reaction, the reaction mixture was cooled, and unreacted triethyl orthoformate and diethyl phosphite were removed by distillation under reduced pressure. The residue was purified by column chromatography on silica gel (chloroform : methanol = 30 : 5) to give tetraethyl(4-benzyl-1-piperazinyl)methylenebisphosphonate (13.8 g).
   MS(DI-EI) m/z 462(M⁺), 325, 187, 91
(ii) To an ethanol solution (200 ml) of the compound (13.0 g) obtained in the above step (i) was added 5% palladium on carbon (2.0 g), and the mixture was subjected to catalytic reduction under ambient pressure. After completion of the reaction, filtration, removal of the ethanol by distillation and purification of the residue by chromatography on silica gel (chloroform : methanol = 30 : 5) afforded tetraethyl (1-piperazinyl)methylenebisphosphonate (9.1 g).
   MS(DI-EI) m/z 372(M⁺), 330, 235, 97
(iii) To a chloroform solution (10 ml) of the compound (1.0 g) obtained in the above step (ii) cooled with ice is added a chloroform solution of trimethylsilyl bromide (2.47 g). After stirring at room temperature for 24 hours an aqueous solution (4 ml) containing sodium acetate (2 g) is added to the reaction mixture and the resulting mixture is stirred for 30 min. The chloroform in the reaction mixture is removed by distillation under reduced pressure followed by crystallization by addition of methanol to produce disodium (1-piperazinyl)methylenebisphosphonate (IX) (0.45 g) represented by the formula shown below.
   MS(FAB) m/z 305(M⁺+1), 283(M⁺-Na+1), 261(M⁺-2Na+1)

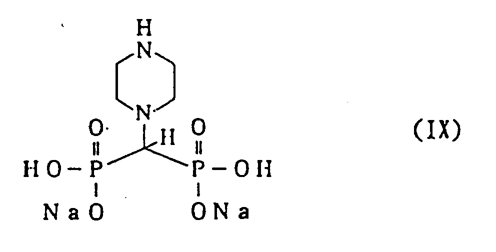

### Example 2

(i) To an ice-cooled solution of 3-[(carboxy-methyl)oxy]-17-(methoxymethoxy)-estra-1,3,5(10)-triene (0.869 g) in dichloromethane (20 ml) is added carbonyl-diimidazole (0.414 g). After stirring the resulting mixture for an hour a solution of tetraethyl [(1-piperazinyl)methylene]bisphosphonate (0.950 g) in dichloromethane (5 ml) is added thereto followed by stirring at room temperature for 3 hours. The reaction mixture is repeatedly purified by column chromatography on silica gel (chloroform : methanol = 9 : 1) to give 3-[[[[4-(bisphosphonomethyl)-1-piperazinyl]carbonyl]methyl]oxy]17-(methoxymethoxy)-estra-1,3,5(10)-trien tetraethyl ester (1.682 g).
   ¹H NMR (89.55 MHz, CDCL₃)
   δ 0.81 (3H, s), 1.33 (12H, t, J=7.0 Hz), 1.05-2.42 (13H, m), 2.55-3.18 (6H, m), 3.35 (3H, s), 3.4-3.75 (6H, m), 3.95-4.40 (8H, m), 4.62 (2H, s), 4.65 (2H, s), 6.65 (1H, s), 6.71 (1H, d, J=8.5 Hz), 7.20 (1H, d, J=8.5 Hz)
(ii) To an ice-cooled chloroform solution (20 ml) of the compound (1.68 g) obtained in the above step (i) is added a solution of trimethylsilyl bromide (2.12 g) in chloroform (5 ml). After stirring the reaction mixture for 24 hours at room temperature, water (2 ml) is added thereto and the resulting mixture is stirred for 30 min. The reaction mixture is concentrated under reduced pressure and the residue is crystallized by addition of a methanol-acetone mixture to afford 3-[[[[4-bisphosphonomethyl)-1-piperazinyl]carbonyl]-methyl]oxy]-estra-1,3,5(10)-trien-17-ol (X) (1.05 g). 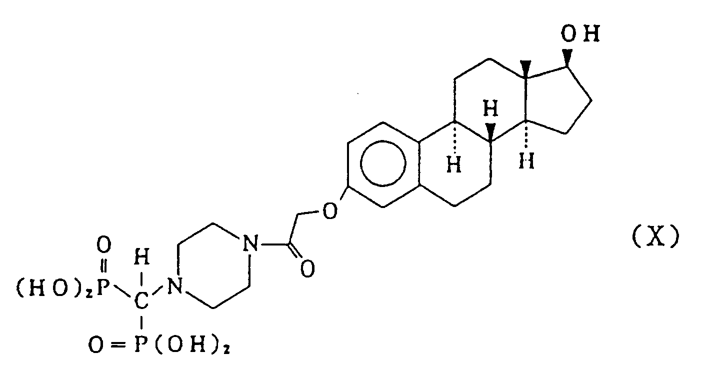 MS(FAB) m/z 573(M⁺+1), 491
   ¹H NMR (500.2 MHz, D₂O+NaHCO₃)
   δ 0.693 (3H, s), 1.05-1.51 (7H, m), 1.642 (1H, m), 1.77-1.90 (2H, m), 1.96-2.08 (2H, m), 2.251 (1H, m) 2.760 (2H, m), 3.323 (1H, t, J=18.1 Hz), 3.57-3.73 (5H, m), 3.829 (4H, m), 4.868 (2H, s), 6.713 (1H, d, J=2 Hz), 6.767 (1H, dd, J=8.8, 2 Hz), 7.256 (1H, d, J=8.8 Hz).

### Example 3

(i) To an ice-cooled solution of 17-(methoxymethoxy)-estra-1,3,5(10)-trien-3-ol (1.0 g) in a mixture of tetrahydrofuran (20 ml) and dichloromethane (30 ml) are added pyridine (0.30 g) and 4-nitrophenyl chloroformate (0.70 g). After stirring at room temperature for 4 hours the reaction mixture is concentrated under reduced pressure and the residue thus obtained is purified by column chromatography on silica gel (chloroform) to give 3-[[(4-nitrophenoxy)-carbonyl]oxy]-17-(methoxymethoxy)-estra-1,3,5(10)-triene (1.03 g).
   MS(DI-EI) m/z 481(M⁺), 449, 436, 418, 324, 298.
(ii) To an ice-cooled chloroform solution (10 ml) of the compound (0.50 g) obtained in the above step (i) is added a solution of tetraethyl [(1-piperazinyl)methylene]-bisphosphonate (0.387 g) in chloroform (5 ml). After stirring at room temperature for 6 hours the reaction mixture is concentrated under reduced pressure and the residue thus obtained is purified by column chromatography on silica gel (chloroform : methanol = 40 : 2) to give 3-[[[4-(bisphosphonomethyl)-1-piperazinyl]carbonyl]oxy]-17- (methoxymethoxy)-estra-1,3,5(10)-trien tetraethyl ester (0.815 g).
   ¹H NMR (89.55 MHz, CDCl₃)
   δ 0.81 (3H, s), 1.37 (12H, t, J=7.0 Hz), 1.05-2.45 (13H, m), 2.60-2.95 (2H, m), 2.95-3.20 (4H, m), 3.37 (3H, s), 3.45-3.75 (6H, m), 4.0-4.40 (8H, m), 4.66 (2H, s), 6.80 (1H, s), 6.84 (1H, d, J=8.5 Hz), 7.25 (1H, d, J=8.5 Hz).
(iii) To an ice-cooled chloroform solution (10 ml) of the compound (0.815 g) obtained in the above step (ii) is added a solution of the trimethylsilyl bromide (0.815 g) in chloroform (5 ml). After stirring the resulting mixtures at room temperature for 24 hours an aqueous solution (2 ml) containing sodium acetate (1.0 g) is added thereto and the resulting mixture is stirred for 30 min. Removal of the chloroform in the reaction mixture by distillation under reduced pressure and crystallization by addition of methanol afford disodium salt of 3-[[[4-(bisphosphonomethyl)-1-piperazinyl]carbonyl]oxy]-extra-1,3,5(10)-tri-en-17-ol (0.510 g) of the formula (XI) shown below. 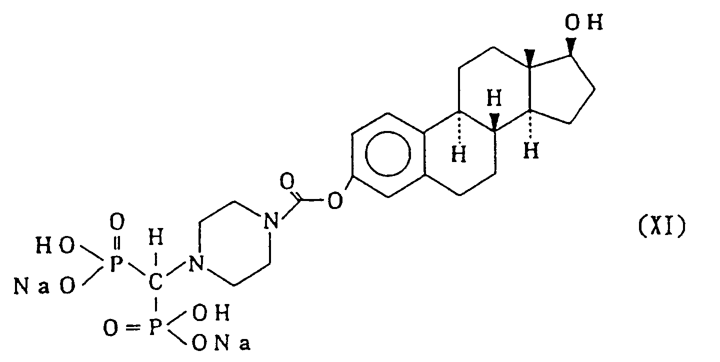 MS(FAB) m/z 603(M⁺+1), 581(M⁺-Na+1), 559(M⁺-2Na+1), 477.
   ¹H NMR (500.2 MHz, D₂O+NaHCO₃)
   δ 0.701 (3H, s), 1.05-1.52 (7H, m), 1.639 (1H, m), 1.809 (1H, m), 1.875 (1H, d-like, J=12.2 Hz), 2.024 (1H, m), 2.095 (1H, m), 2.257 (1H, m), 2.774 (2H, m), 3.381 (1H, t, J=17.6 Hz), 3.675 (1H, t, J=8.5 Hz), 3.744 (4H, brs), 3.819 (2H, brs), 3.983 (2H, brs), 6.853 (1H, d, J=2 HZ), 6.905 (1H, dd, J=8.3, 2 Hz), 7.326 (1H, d, J=8.3 Hz).

## Claims

1. A compound represented by the formula (I) 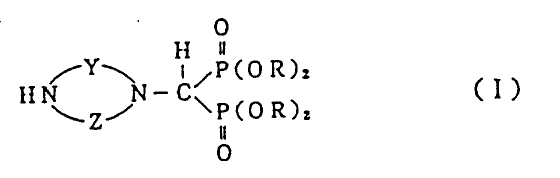 or the formula (II) 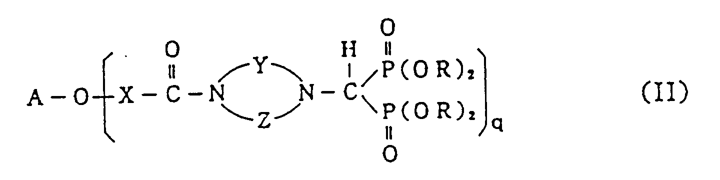 wherein R denotes a hydrogen atom or a lower alkyl group; Y and Z may be the same or different and independently denote alkylene groups containing 2-5 carbon atoms in their alkylene chains which may be substituted with a lower alkyl(s); X denotes a single bond or an alkylene group containing 1-10 carbon atoms in its alkylene chain which may be substituted with a lower alkyl(s); A-O- denotes a residue of a compound having an estrogenic activity and q denotes an integer of 1-3, and physiologically acceptable salts thereof.

2. The compound of claim 1 represented by the formula (III) 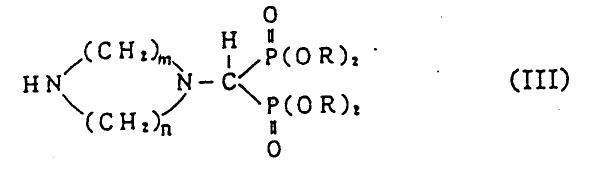 where R has the same meaning as defined above and m and n may be the same or different and independently denote integers of 2 or 3, and physiologically acceptable salts thereof.

3. The compound of claim 2 wherein both m and n are 2 and physiologically acceptable salts thereof.

4. The compound of claim 1 represented by the formula (IV) 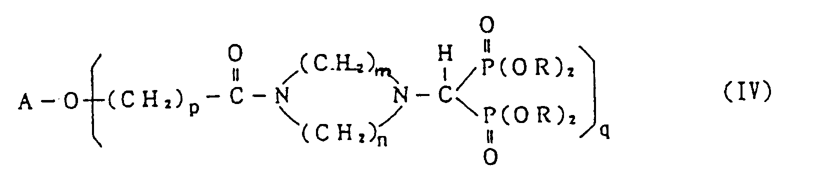 wherein A-O-, R, q, m and n have the same meanings as defined above and p denotes an integer of 0-5, and physiologically acceptable salts thereof.

5. The compound of claim 4 wherein p is 0 or 1 and both m and n are 2 and physiologically acceptable salts thereof.

6. A pharmaceutical composition for inhibiting a bone resorption comprising an effective amount of the compound or physiologically acceptable salt thereof according to claims 1-5 in combination with a pharmaceutically acceptable carrier or excipient.

7. The use of an effective amount of the compound or physiologically acceptable salt thereof according to claims 1 to 5 for the production of a pharmaceutical for treating bone diseases or inhibiting a bone resorption.

8. A pharmaceutical composition containing an effective amount of the compound or physiologically acceptable salt thereof according to claims 1 to 5 in combination with a pharmaceutically acceptable carrier or excipient.
